# EUROPEAN PATENT SPECIFICATION

(11) **EP 4 223 289 B1**
(45) Date of publication and mention of the grant of the patent: **18.06.2025**
(21) Application number: 22163428.0
(22) Date of filing: 22.03.2022
(51) Int. Cl.: A61K 31/18, A61K 31/713, A61P 17/06, A61K 31/138, A61K 31/135, A61K 31/137

(54) **MEDICAMENT COMPRISING THE ADRB2 INHIBITOR ICI 118551 FOR USE IN TREATING PSORIASIS**
ARZNEIMITTEL, DAS DEN ADRB2-INHIBITOR ICI 118551 ENTHÄLT, ZUR BEHANDLUNG VON PSORIASIS
MÉDICAMENT COMPRENANT L'INHIBITEUR DE L'ADRB2 ICI 118551 POUR LE TRAITEMENT DU PSORIASIS

(30) Priority: 07.02.2022 CN 202210116648
(43) Date of publication of application: 09.08.2023
(73) Proprietor: Sun, Liangdan, Hefei City, Anhui 230022 (CN)
(72) Inventor: SUN, Liangdan, Hefei City, Anhui (CN); YU, Yafen, Hefei (CN); YONG, Liang, Hefei (CN); ZHEN, Qi, Hefei (CN); Li, BAO, Hefei (CN)
(74) Representative: Herrero & Asociados, S.L.

(56) References cited:
- WO-A1-2020/144538
- WO-A1-2021/004229
- CA-A1- 2 708 613
- MASON J ET AL: "Topical preparations for the treatment of psoriasis: a systematic review", BRITISH JOURNAL OF DERMATOLOGY, JOHN WILEY, HOBOKEN, USA, vol. 142, no. 3, 28 June 2008 (2008-06-28), pages 351 - 364, XP071116330, ISSN: 0007-0963, DOI: 10.1046/J.0007-0963.2002.04713.X
- ZABA L C ET AL: "Effective treatment of psoriasis with etanercept is linked to suppression of IL-17 signaling, not immediate response TNF genes", JOURNAL OF ALLERGY AND CLINICAL IMMUNOLOGY, ELSEVIER, AMSTERDAM, NL, vol. 124, no. 5, 1 November 2009 (2009-11-01), pages 1022, XP026732722, ISSN: 0091-6749, [retrieved on 20091104], DOI: 10.1016/J.JACI.2009.08.046
- XIE XIANG-JIANG ET AL: "Indirubin ameliorates imiquimod-induced psoriasis-like skin lesions in mice by inhibiting inflammatory responses mediated by IL-17A-producing [gamma][delta] T cells", MOLECULAR IMMUNOLOGY, PERGAMON, GB, vol. 101, 29 July 2018 (2018-07-29), pages 386 - 395, XP085470441, ISSN: 0161-5890, DOI: 10.1016/J.MOLIMM.2018.07.011
- NADEEM AHMED ET AL: "Bruton's tyrosine kinase inhibitor suppresses imiquimod-induced psoriasis-like inflammation in mice through regulation of IL-23/IL-17A in innate immune cells", INTERNATIONAL IMMUNOPHARMACOLOGY, ELSEVIER, AMSTERDAM, NL, vol. 80, 24 January 2020 (2020-01-24), XP086048878, ISSN: 1567-5769, [retrieved on 20200124], DOI: 10.1016/J.INTIMP.2020.106215
- RIOL-BLANCO LORENA ET AL: "Nociceptive sensory neurons drive interleukin-23-mediated psoriasiform skin inflammation", NATURE, vol. 510, no. 7503, 23 April 2014 (2014-04-23), London, pages 157 - 161, XP055970126, ISSN: 0028-0836, Retrieved from the Internet <URL:http://www.nature.com/articles/nature13199> DOI: 10.1038/nature13199
- ABEL E A ET AL: "Drugs in exacerbation of psoriasis", JOURNAL OF THE AMERICAN ACADEMY OF DERMATOLOGY, MOSBY, INC, US, vol. 15, no. 5, 1 November 1986 (1986-11-01), pages 1007 - 1022, XP024559653, ISSN: 0190-9622, [retrieved on 19861101], DOI: 10.1016/S0190-9622(86)70265-X
- BAKER FORREST L. ET AL: "Systemic [beta]-Adrenergic Receptor Activation Augments the ex vivo Expansion and Anti-Tumor Activity of V[gamma]9V[delta]2 T-Cells", FRONTIERS IN IMMUNOLOGY, vol. 10, 24 January 2022 (2022-01-24), XP055971409, DOI: 10.3389/fimmu.2019.03082
- LIU FEI ET AL: "(R)-Salbutamol Improves Imiquimod-Induced Psoriasis-Like Skin Dermatitis by Regulating the Th17/Tregs Balance and Glycerophospholipid Metabolism", CELLS, vol. 9, no. 2, 24 February 2020 (2020-02-24), pages 511, XP055971450, DOI: 10.3390/cells9020511
- LI YONG-LIANG ET AL: "Aromatic-turmerone ameliorates imiquimod-induced psoriasis-like inflammation of BALB/c mice", INTERNATIONAL IMMUNOPHARMACOLOGY, ELSEVIER, AMSTERDAM, NL, vol. 64, 19 September 2018 (2018-09-19), pages 319 - 325, XP085497651, ISSN: 1567-5769, DOI: 10.1016/J.INTIMP.2018.09.015

## Description

### TECHNICAL FIELD

The invention relates to the field of psoriasis medicine technologies, in particular, to a medicament for use in a method of treating psoriasis, comprising a beta 2 adrenergic receptor (ADRB2) inhibitor, wherein the ADRB2 inhibitor is ICI 118551. The invention is set out in the appended claim.

### BACKGROUND

Psoriasis is a chronic inflammatory skin disease characterized by immunocyte infiltration, epidermal hyperplasia and abnormal differentiation of keratinocytes. Previous studies have found that keratinocytes and immunocytes interact to produce cytokine networks, especially interleukin-17 (IL-17) family and its subsequent signal cascade, driving the development of psoriasis. The polygenic inheritance of psoriasis reveals that the occurrence and development of psoriasis may be related to various factors such as inflammation, cell proliferation and apoptosis, neuropsychiatric factors. How genetic susceptibility affects the psoriasis progression, especially which cell types are involved in psoriasis-specific genetic alterations, remains unknown. Previous genome-wide association studies (GWAS) and meta-analyses have found a variety of psoriasis susceptibility genes, and these studies on the variation of psoriasis susceptibility genome have also repeatedly suggested some correlations between psoriasis susceptibility genes and disease phenotype, risk and mechanism. For instance, genotype-phenotype association studies have found that rs10852936 is associated with early-onset psoriasis (onset age < 40 years), rs72474224 (GJB2) is associated with clinical features of psoriasis vulgaris (also referred to plaque psoriasis), and rs1020760 (NFKB1) is associated with psoriasis having a positive family history. Gene-gene interactions (e.g., MHC-LCE interaction, MHC-IL12B interaction) can increase the risk of psoriasis. Susceptibility genes are associated with a variety of psoriasis co-morbidities or other immune diseases. For example, by exploring the common susceptibility sites of psoriasis and SLE, it is found that NFKBIA and IL28RA reached genome-wide association significance in the two diseases. Some functional studies have found that many susceptibility genes are located near genes related to specific adaptive and innate immune pathways, including antigen presentation (HLA-C, ERAP1), IL-17/23 axis activation (IL23R, IL23A, IL12B, TRAF3IP2), innate antiviral immunity/type I interferon signaling (RNF114, IFIH1) and skin barrier function (LCE3B/3D).

Psoriasis, as a complex disease, is not affected by a single factor. Finding the internal occurrence and regulation mechanism of the disease and the weight of various influencing factors in the disease is the basis and prerequisite for the efficient selection of disease targets in the future. As an upstream of disease research, genomic variation is a source of confirmation for the study of disease pathogenesis, but the research on downstream mechanisms of many susceptibility genes currently found is still lacking. Although some suggestive results related to disease characteristics can often be obtained through gene association analysis, the level is mostly limited to the preliminary judgment on a direction of gene function, and lacks of analysis on the mechanism of susceptibility genes in the pathogenesis of psoriasis at levels of proteins, cell animal models and tissue samples. Without being able to determine the pathogenesis of psoriasis, it is impossible to provide effective agents for psoriasis.

Xie et al., 2018, Molecular Immunology, 101:386-395 discloses that indirubin alleviates IMQ-induced psoriasis-like dermatitis mainly through reducing the inflammatory responses mediated by IL-17A-producing γδ T cells involving Jak3/Stat3 activation. Our results highlighted the novel mechanisms by which Indirubin ameliorates psoriasis-related inflammatory responses, supporting its therapeutic potential.

Abel et al., 1986, Journal of the American Academy of Dermatology, 15(5):1007-1022 discloses that drugs that have been associated with the precipitation or exacerbation of psoriasis include lithium, beta adrenergic receptor blocking agents, and antimalarials. The withdrawal of corticosteroids has been reported to activate pustular psoriasis. Nonsteroidal anti-inflammatory drugs, such as indomethacin, have recently been reported to exacerbate psoriasis, although additional well-controlled studies are still needed. Drugs used for treatment of psoriasis will sometimes cause a flare because of irritation, phototoxicity, or hypersensitivity reaction resulting in a Koebner phenomenon. Because psoriasis is a very complex disease and its activity is often unpredictable, clinical studies on adverse drug effects on psoriasis have been difficult to conduct. This review evaluates clinical, histologic, and biochemical evidence in the literature for drug-associated onset or exacerbation of psoriasis.

Baker et al., 2022, Frontiers in Immunology, 10:3389 discloses that TCR-gamma delta (γδ) T-cells are considered important players in the graft-vs.-tumor effect following allogeneic hematopoietic cell transplantation (alloHCT) and have emerged as candidates for adoptive transfer immunotherapy in the treatment of both solid and hematological tumors, Systemic β-adrenergic receptor (β-AR) activation has been shown to mobilize TCR-γδ T-cells to the blood, potentially serving as an adjuvant for alloHCT and TCR--γδ T-cell therapy. We investigated if systemic β-AR activation, using acute dynamic exercise as an experimental model, can increase the mobilization, *ex vivo* expansion, and anti-tumor activity of TCR-γδ T-cells isolated from the blood of healthy humans. We also sought to investigate the β-AR subtypes involved, by administering a preferential β-AR antagonist (bisoprolol) and a non-preferential β₁ + β₂-AR antagonist (nadolol) prior to exercise as part of a randomized placebo controlled cross-over experiment.

Liu et al., 2020, Cells, 9(2):511 discloses that psoriasis is a skin disease that is characterized by a high degree of inflammation caused by immune dysfunction. (R)-salbutamol is a bronchodilator for asthma and was reported to alleviate immune system reactions in several diseases. In this study, using imiquimod (IMQ)-induced mouse psoriasis-like dermatitis model, we evaluated the therapeutic effects of (R)-salbutamol in psoriasis in vivo, and explored the metabolic pathway involved. The results showed that, compared with IMQ group, (R)-salbutamol treatment significantly ameliorated psoriasis, reversed the suppressive effects of IMQ on differentiation, excessive keratinocyte proliferation, and infiltration of inflammatory cells. Enzyme-linked immunosorbent assays (ELISA) showed that (R)-salbutamol markedly reduced the plasma levels of IL-17. Cell analysis using flow cytometry showed that (R)-salbutamol decreased the proportion of CD4+ Th17+ T cells (Th17), whereas it increased the percentage of CD25+ Foxp3+ regulatory T cells (Tregs) in the spleens. (R)-salbutamol also reduced the increased weight ratio of spleen to body. Furthermore, untargeted metabolomics showed that (R)-salbutamol affected three metabolic pathways, including (i) arachidonic acid metabolism, (ii) sphingolipid metabolism, and (iii) glycerophospholipid metabolism. These results demonstrated that (R)-salbutamol can alleviate IMQ-induced psoriasis through regulating Th17 /Tregs cell response and glycerophospholipid metabolism.

### SUMMARY

In view of this, on a basis of genetics, susceptibility sites really and significantly related to psoriasis are found out, and a variety of functional technologies are used to analyze an action mechanism of a susceptibility gene calcium/calmodulin-dependent protein kinase type II gamma (CAMK2G) in pathogenesis of psoriasis at levels of proteins, cell animal models and tissue samples. The invention is set out in the appended claim.

Disclosed herein, but not claimed, is an application of a preparation for inhibiting CAMK2G expression in preparation of a medicine for treating psoriasis.

For example, the CAMK2G expression includes at least one from a group consisting of CAMK2G transcription and messenger ribonucleic acid (mRNA) translation after the CAMK2G transcription.

Specifically, a CAMK2G transcription level in peripheral blood of psoriasis patients is significantly higher than that of healthy controls, and the CAMK2G transcription level in peripheral blood of psoriasis mice is also higher than that of a control group. A proportion of CAMK2G+ cells in the peripheral blood of psoriasis patients is significantly higher than that of the healthy controls.

Disclosed herein, but not claimed, is an application of a preparation for a CAMK2y protein activity in preparation of a medicine for treating psoriasis.

Specifically, a total protein level and a phosphorylated active protein level of CAMK2y of in skin of the psoriasis mice are higher than those in the control group, and a total protein level and a phosphorylated active protein level of CAMK2y of the psoriasis patients are significantly higher than in the healthy controls.

Disclosed herein, but not claimed, is an application of a preparation for inhibiting release of neutrophil extracellular traps (NETs) by neutrophils in preparation of a medicine for treating psoriasis.

Specifically, a mean fluorescence intensity (MFI) CAMK2y of neutrophils in the psoriasis patients is significantly higher than that of the healthy controls, and there is no significant difference in monocytes cluster of differentiation 14+ (CD14+) and multiple T cell subsets. Cells with high CaMK2y expression in a circulatory system mainly are neutrophils. A transcription level of CAMK2G in blood neutrophils of the psoriasis patients is significantly higher than that of the healthy controls.

Disclosed herein, but not claimed, is an application of a preparation for removing yδ T cells or inhibiting growth of γδ T cells in preparation of a medicine for treating psoriasis.

For example, the γδ T cells are dermal γδ T cells.

Specifically, interluekin-17A (IL-17A) is mainly produced by dermal γδ T cells in dorsal skin of imiquimod (IMQ)-treated wild type (WT) mice, and rarely expressed in epidermal γδ T cells and αβ T cells. In IMQ-treated *Camk2g*^{*-*/*-*} mice, IL-17A+ dermal γδ T cells are significantly lower than the IMQ-treated WT mice, while IL-17A+ epidermal yδ T cells and IL-17A+ αβ T cells remained unchanged.

Specifically, γδ T cell deficient-mice (*Tcrd*^{*-*/*-*}) are injected subcutaneously with salmeterol xinafoate (SAL) and promotion of psoriatic phenotype and inflammatory infiltration by SAL is counteracted by lack of γδ T cells.

Disclosed herein, but not claimed, is an application of a preparation for removing or inhibiting a sympathetic nerve activity of skin tissue in preparation of a medicine for treating psoriasis.

For example, the preparation for removing or inhibiting the sympathetic nerve activity of skin tissue is 6-hydroxydopamine (6-OHDA).

Specifically, CAMK2γ in skin tissue is mainly expressed in sympathetic nerve fibers, and there are more CaMK2y+ sympathetic nerves in the skin of psoriasis patients than in healthy skin. Removal of skin sympathetic nerve reduces IMQ-induced psoriasis-like phenotype in mice. The decrease of IL-17A+ γδ T cells in the skin without the sympathetic nerves indicates that the skin sympathetic nerves affect psoriasis-like skin inflammation by regulating γδ T cells to produce IL-17.

Specifically, 6-OHDA-induced injury in SH-SY5Y cells can lead to a decrease in intracellular tyrosine hydroxylase (TH) transcription level, and knockout of the CAMK2G gene can also reduce the TH transcription level; 6-OHDA-induced injury in SH-SY5Y cells has no significant effect on the expression of CAMK2G.

Disclosed herein, but not claimed, is an application of a preparation for inhibiting norepinephrine secretion by sympathetic nerves in skin tissue in preparation of a medicine for treating psoriasis.

Specifically, subcutaneous injection of norepinephrine (NE) exacerbates the IMQ-induced psoriasis-like phenotype and increases a proportion of IL-17A+ γδ T cells in mouse skin, and more importantly, administration of NE counteracted the decrease in IL-17A+ γδ T cells caused by CAMK2G deletion. After NE injection, differences in skin thickness between IMQ-induced WT mice and IMQ-induced *Camk2g*^{*-*/*-*} mice disappeared.

Disclosed herein, but not claimed, is an application of a preparation for inhibiting production or growth of tyrosine hydroxylase (TH) in skin tissue in preparation of a medicine for treating psoriasis.

Specifically, the significant reduction of TH and phosphorylated tyrosine hydroxylase (pTH) confirms inactivation of local sympathetic nerves in the skin tissue.

The invention aims to provide a medicament for use in a method of treating psoriasis, comprising a beta 2 adrenergic receptor (ADRB2) inhibitor, wherein the ADRB2 inhibitor is ICI 118551.

Specifically, in some specific embodiments, psoriasis phenotype of mice injected with ICI is reduced; in IMQ-induced mouse skin, injection of the ICI reduces a proportion of skin γδ T-17 cells.

Disclosed herein, but not claimed, is an application of a preparation for inhibiting p38 (also referred to as p38 mitogen-activated protein kinase (p38 MAPK)) phosphorylation in preparation of a medicine for treating psoriasis.

Specifically, SAL treatment enhances activation of p38 in IMQ-induced skin, while ICI treatment showed inhibitory effect. The p38 phosphorylation is inhibited in the skin of *Camk2g*^{*-*/*-*} mice, and NE injection increases and restores the p38 phosphorylation.

The invention discloses the following technical effects:

The preparations of the present disclosure can effectively inhibit the abnormal activation of CAMK2G in the skin sympathetic nerves to stimulate IL-17 secretion of γδ T cells, so as to reduce the immune response mediated by IL-17 in psoriatic skin and achieve the effect of treating psoriasis. It can also reduce production of neutrophil NETs in a circulatory system and inhibit aggravation of the disease, so as to have the effect of treating immune deficiency diseases.

### BRIEF DESCRIPTION OF DRAWINGS

FIG. 1 is a design flowchart of the present disclosure.
FIG. 2A shows a calcium/calmodulin-dependent protein kinase type II gamma (CAMK2G) transcription level in peripheral blood of psoriasis patients.
FIG. 2B shows a proportion of CAMK2G+ cells in peripheral blood of psoriasis patients.
FIG. 2C shows a CAMK2G transcription level of in peripheral blood of psoriasis mice.
FIG. 2D shows a total protein level and a phosphorylated active protein level of CAMK2y in skin tissue of psoriasis mice.
FIG. 3A shows CAMK2y expression in cutaneous nerves.
FIG. 3B shows a condition of CAMK2y+ sympathetic nerves in skin of psoriasis patients.
FIGS. 4A-4B show a condition of cells with high expression of CAMK2y in a circulatory system.
FIGS. 5A-5D show CAMK2y mean fluorescence intensity (MFI) of neutrophils from psoriasis patients.
FIG. 6 shows a transcription level of CAMK2G in blood neutrophils of psoriasis patients.
FIG. 7 shows a total protein level of CAMK2y and a phosphorylated protein level of CAMK2y in blood neutrophils of psoriasis patients.
FIG. 8 shows hematoxylin and eosin (H&E) staining of skin sections of *Camk2g^{-l-}* mice.
FIGS. 9A-9D show psoriasis area severity index (PASI) scores of *Camk2g^{-l-}* mice.
FIGS. 10A-10D show typical inflammatory infiltrating cells in psoriatic lesions by flow cytometry.
FIG. 11 shows typical inflammatory infiltrating cells in dorsal skin of *Camk2g^{-l-}* mice induced by imiquimod (IMQ).
FIG. 12 shows expression of inflammatory factor gene in the skin of *Camk2g^{-l-}* mice.
FIGS. 13A-13C show interleukin-17A (IL-17A) expression cells in dorsal skin of IMQ-treated wild type (WT) mice by flow cytometry.
FIGS. 14A-14C show different mouse model cells by flow cytometry.
FIG. 15 shows IL-17A+ dermal γδ T cell of IMQ-treated *Camk2g*^{*-*/*-*} mice.
FIG. 16 shows IL-17A+ epidermal γδ T cell of IMQ-treated *Camk2g*^{*-*/*-*} mice.
FIG. 17 shows IL-17A+ αβ T cells of IMQ-treated *Camk2g*^{*-*/*-*} mice.
FIG. 18 shows TH phosphorylation in the skin of IMQ-treated *Camk2g*^{*-*/*-*} mice.
FIG. 19 shows norepinephrine (NE) levels in the skin of IMQ-treated *Camk2g^{-l-}* mice.
FIG. 20 shows dopamine levels in the skin of IMQ-treated *Camk2g^{-l-}* mice.
FIG. 21 is test flowchart of psoriasis-like phenotype induced by subcutaneous injection of NE and IMQ in mice.
FIG. 22 shows a proportion of IL-17A+ γδ T cells in mouse skin after subcutaneous injection of NE.
FIG. 23 shows changes in IL-17A+ γδ T cells in mice with CAMK2G deficiency after subcutaneous injection of NE.
FIG. 24 shows skin thickness of IMQ-induced WT mice and IMQ-induced *Camk2g*^{*-*/*-*} mice after subcutaneous injection of NE.
FIG. 25 is test flowchart of mice intradermally injected with 6-hydroxydopamine (6-OHDA) before IMQ treatment.
FIG. 26 shows distribution of sympathetic nerves in mouse skin after intradermal injection of 6-OHDA by immunofluorescence.
FIG. 27 shows expression of TH and pTH in mice after intradermal injection of 6-OHDA by western blot.
FIG. 28 shows skin phenotype of mice after intradermal injection of 6-OHDA.
FIGS. 29A-29D show psoriasis-like phenotype of mice after intradermal injection of 6-OHDA.
FIGS. 30A-30C show IL-17A+ γδ T cells in mouse skin after intradermal injection of 6-OHDA.
FIG. 31 shows NE in mouse skin after intradermal injection of 6-OHDA.
FIG. 32 shows effects of SH-SY5Y cell injury induced by 6-OHDA on a transcription level of intracellular TH.
FIG. 33 shows effects of SH-SY5Y cell injury induced by 6-OHDA on CAMK2G expression.
FIG. 34 shows effects of SH-SY5Y cell injury induced by 6-OHDA on a total protein and a phosphorylation level of CAMK2G.
FIG. 35 shows RNA sequencing of skin tissue of IMQ-induced psoriasis mice.
FIG. 36 shows immunohistochemical analysis of skin lesions in psoriasis patients and healthy controls.
FIG. 37 shows expression of β2-AR by IL-17 expressing cells.
FIGS. 38A-38B show a proportion of β2-AR+ T cells in muse skin after IMQ treatment.
FIG. 39 is test flowchart of IMQ-induced mice injected with salmeterol xinafoate (SAL).
FIG. 40 shows skin of IMQ-induced mice injected with SAL.
FIGS. 41A-41D show thickness, erythema and scaling of dorsal skin of IMQ-induced mice injected with SAL.
FIGS. 42A-42C show IL-17A production in IMQ-induced mice injected with SAL.
FIG. 43 is a test flowchart of IMQ-induced mice injected subcutaneously with ICI.
FIG. 44 shows skin of IMQ-induced mice injected subcutaneously with ICI.
FIGS. 45A-45D show thickness, erythema and scaling of dorsal skin of IMQ-induced mice injected subcutaneously with ICI.
FIGS. 46A-46C show a proportion of γδT-17 cells in IMQ-induced mice injected subcutaneously with ICI.
FIG. 47 shows skin phenotype of γδ T cell-deficient mice (*Tcrd*^{*-*/*-*}) after subcutaneous injection of SAL.
FIG. 48 shows a proportion of neutrophils in the skin after subcutaneous injection of SAL of γδ T cell-deficient mice (*Tcrd*^{*-*/*-*}).
FIG. 49 shows a proportion of monocytes in the skin after subcutaneous injection of SAL in yδ T cell-deficient mice (*Tcrd*^{*-*/*-*}).
FIG. 50 shows IL-17 production by αβ T cells after subcutaneous injection of SAL in γδ T cell-deficient mice (*Tcrd*^{*-*/*-*}).
FIGS. 51A-51C show T cell pro-inflammatory cytokine production under synergistic stimulation with Phorbol 12-myristate 13-acetate (PMA) and ionomycin (Ion).
FIGS. 52A-52C show T cell pro-inflammatory cytokine production stimulated by ICI.
FIG. 53 shows activation of p38, extracellular signal-regulated kinase (ERK) and c-Jun N-Terminal Kinase (JNK) in PMA/ion activated Jurkat T cells.
FIG. 54 shows p38 phosphorylation in IMQ-induced skin after SAL treatment.
FIG. 55 shows p38 phosphorylation in IMQ-induced skin after ICI treatment.
FIG. 56 shows p38 phosphorylation in IMQ-treated WT mice.
FIGS. 57A-57C show inhibition of myeloperoxidase (MPO), elastase and peptidyl arginine deiminase 4 expression by CaMKII-IN1 and KN93 in PMA-activated HL-60 cells.
FIG. 58 shows a total number of intracellular ROS under inhibition of CAMK2y activity and.
FIGS. 59A-59C show mRNA levels of NCF1, NCF2 and NOX2 under the inhibition of CAMK2y activity. The above-mentioned FIGS. 1-34 and 57A-59C are not according to the invention and are present for illustration purposes only.

### DETAILED DESCRIPTION OF EMBODIMENTS

Embodiments are intended to illustrate the invention better, but the content of the invention is not limited to the embodiments. Therefore, non-essential changes and adjustments of the implementation solutions by those skilled in the art according to the invention still belong to the protection scope of the invention.

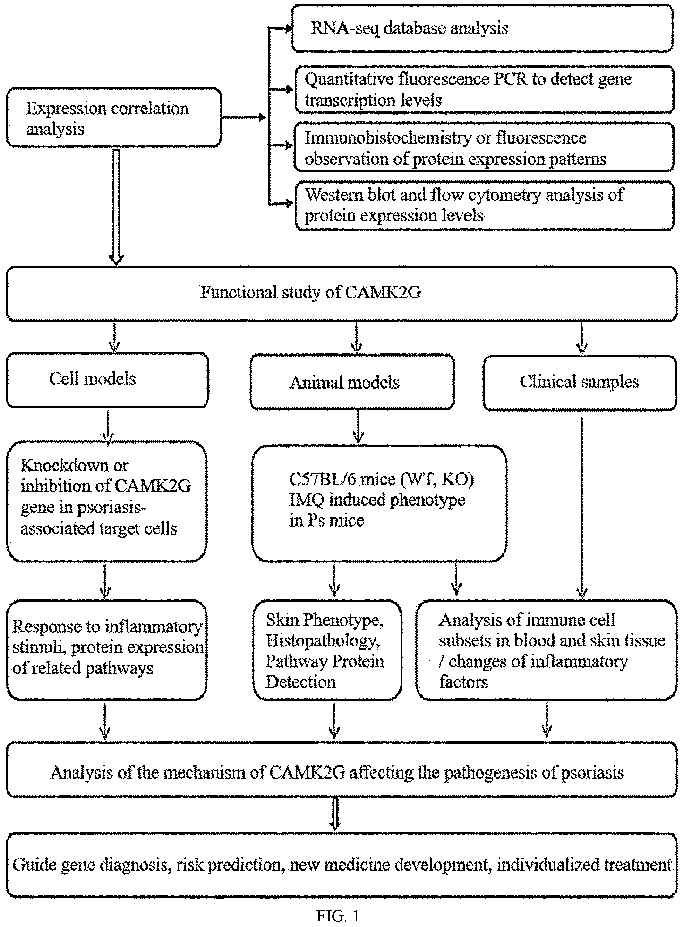

In the present disclosure, experimental designs are carried out according to a design flowchart shown in FIG. 1.

In the present disclosure, IMQ refers to imiquimod, which is an agonist of Toll-like receptor (TLR) 7 and TLR 8.

In the present disclosure, the term "agonist" refers to a substance or drug that binds to a receptor of a bioactive substance and exhibits effects of the active substance.

In the present disclosure, the term "antagonist" refers to a kind of substance that can bind to receptors but are not intrinsically active. Antagonists are divided into competitive antagonists and noncompetitive antagonists.

In the present disclosure, "Phorbol 12-myristate 13-acetate (PMA)" and "ionomycin (Ion)" are used to activate Jurkat cells, and only activated Jurkat cells can highly express inflammatory factors.

In the present disclosure, "CAMK2γ" is one of protein products expressed by calcium/calmodulin-dependent protein kinase type II gamma (CAMK2G) gene.

In the embodiments of the invention, "CAMK2G+ cells" refers to cells expressing CAMK2G protein.

In the present disclosure, "IMQ-induced *Camk2g^{-l-}* psoriasis mouse model" refers to CAMK2G gene knockout mice induced by IMQ, and "IMQ-induced wild type mouse model" refers to wild type mice induced by IMQ. The following examples 1-8 and 15 are not according to the invention and are present for illustration purposes only.

### Example 1 expression detection of susceptible gene CAMK2G in psoriasis

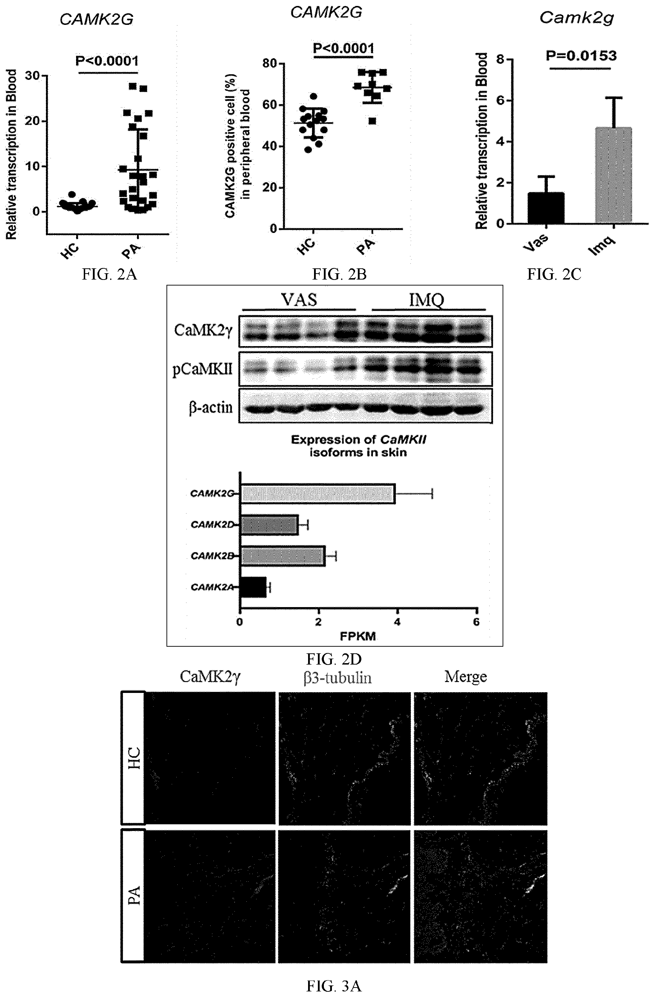

In order to detect a correlation between the expression of susceptible gene CAMK2G and psoriasis, the expression of CAMK2G in the blood and skin tissues of psoriasis patients and psoriasis mice are detected respectively. Results detected by real-time quantitative polymerase chain reaction (RT-qPCR) and flow cytometry showed that transcription level of CAMK2G in peripheral blood of psoriasis patients is significantly higher than that of healthy controls (FIG. 2A). A proportion of CAMK2G+ cells in peripheral blood of psoriasis patients is significantly higher than that of the healthy controls (FIG. 2B). The transcription level of CAMK2G in peripheral blood of psoriasis mice is higher than that in the control group (FIG. 2C).

The existing literature discloses that CAMKII γ plays the effect of second messenger in many Ca²⁺-mediated signaling pathways. When intracellular Ca²⁺ is at basal level, its self-inhibition is maintained in a dormant state. When intracellular Ca²⁺ level increases, it undergoes autophosphorylation to release the inhibitory state, and phosphorylation at Thr287 is an active form of CAMK2y.

There is currently a lack of phosphorylated CaMK2y-specific antibodies on the market, however, mouse skin RNA-seq results show that CAMK2y is the most highly expressed member of the CAMKII family in skin tissue. Therefore, phospho-CAMKII (Thr287) antibody is used to detect the activity of CAMK2γ in skin tissue. Western blot detection shows that a total protein level and a phosphorylated active protein level of CAMK2y in the skin tissue of psoriasis mice are higher than those of the control group (FIG. 2D).

### Example 2 Expression and localization of CAMK2γ in skin tissue

In order to verify whether CAMK2G is highly expressed in nerve cells in skin tissue, skin samples from healthy controls and psoriasis patients are co-stained for CAMK2y and pan-neuronal marker β3-tubulin, and immunofluorescence results shows that CAMK2y is expressed in cutaneous nerves, but not all nerve cells are positive for CAMK2y (FIG. 3A). That is, members of the CAMKII family are highly expressed in nerve cells, and cutaneous nerves are composed of sympathetic nerve fibers and several types of sensory nerve fibers.

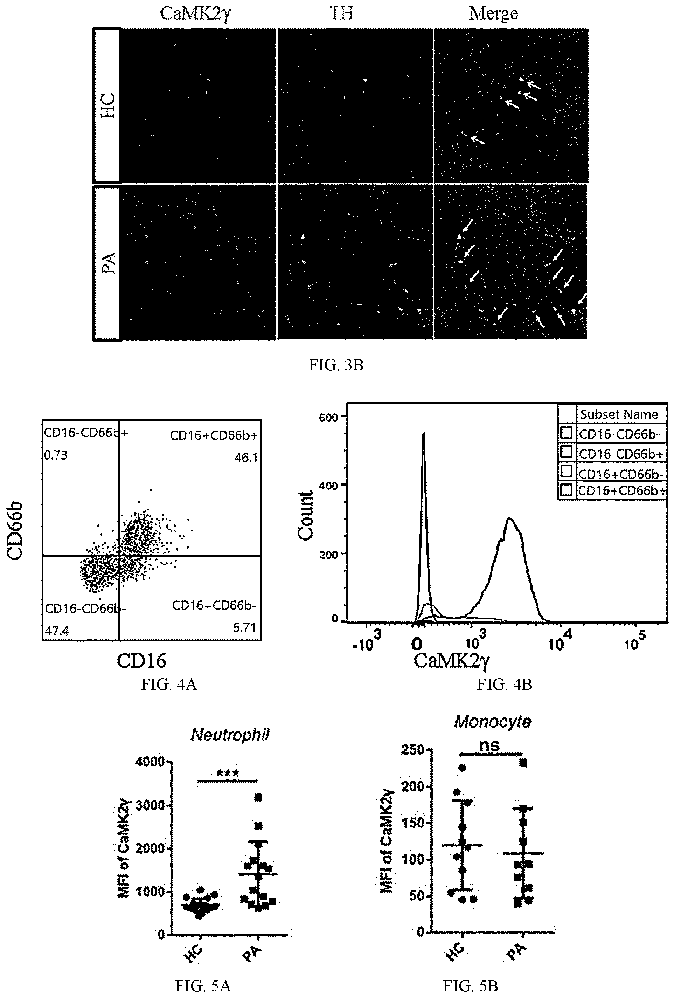

Immunofluorescence co-staining is further performed with tyrosine hydroxylase (TH) which recognizes sympathetic nerve fibers and CAMK2γ, and immunofluorescence results shows CAMK2y in skin tissue is mainly expressed in the sympathetic nerve fibers, and there are more CAMK2y+ sympathetic nerves in the skin tissue of psoriasis patients than in healthy skin tissue (FIG. 3B).

### Example 3 Expression and localization of CAMK2γ in circulatory system

Mean fluorescence intensity (MFI) of CAMK2y in CD16+CD66b+ neutrophils and non-neutrophils including CD16+CD66b- cells and CD16-CD66b- cells in human peripheral blood is analyzed by flow cytometry, and analysis results shows that cells with high expression of CAMK2y in the circulatory system are mainly the neutrophils (FIGS. 4A-4B).

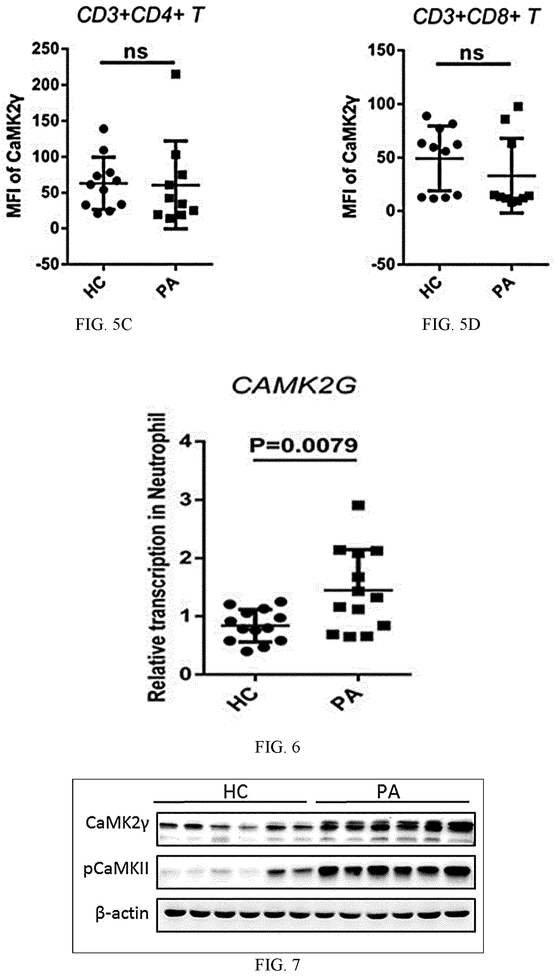

In order to further detect changes of CAMK2y in several representative immune cells in peripheral blood of psoriasis patients, different immune cell subsets in peripheral blood of psoriasis patients and healthy controls are analyzed by flow cytometry, and analysis results shows that the MFI of CAMK2y in neutrophils of psoriasis patients is significantly higher than that in healthy controls, while there is no significant difference in monocytes (CD14+) and multiple T cell subsets (FIGS. 5A-5D).

In order to further detect the change of CAMK2G expression in neutrophils in the circulatory system of psoriasis patients, high-purity neutrophils are separated from the blood of psoriasis patients and healthy controls by magnetic bead sorting, and results detected by RT-qPCR shows that the transcription level of CAMK2G in blood neutrophils of psoriasis patients is significantly higher than that of healthy controls (P = 0.0079) (FIG. 6). Western blot analysis shows that both the total protein level and the active form of phosphorylation of CAMK2y are significantly higher in psoriasis patients than in healthy controls (FIG. 7).

### Example 4 Establishment of IMQ-induced Camk2g^{-/-} psoriasis a mouse model

In order to further study an action mechanism of CAMK2G in psoriasis, the IMQ-induced *Camk2g^{-l-}* psoriasis mouse model is established.

Establishment steps of IMQ-induced *Camk2g*^{*-*/*-*} psoriasis mouse model include steps as follows. 7-week-old wild type mice (WT) (n = 5) or *Camk2g*^{*-*/*-*} mice (n = 5) are taken to shave back hair, and the exposed skin area is 2 centimeters (cm) × 2 cm, 62.5 micrograms (mg) of IMQ cream or control agent Vaseline (VAS) are evenly applied to the exposed skin of mice for 4 consecutive days, psoriasis area severity index (PASI) score is performed every day, and the mice are sacrificed on the 5th day to take the skin, spleen and blood for testing.

The skin tissue is fixed with 10% formalin, embedded in paraffin, cut into 4 micrometers (µm) sections for hematoxylin and eosin (H&E) staining.

H&E stained images are obtained using an upright microscope (Olympus BX53) in a 10-fold (×100) field of view.

The inflammation scoring method of the mouse model is an objective scoring system developed on the basis of clinical PASI score. Scores of erythema, scaling and thickening ranged from 0 to 4:0: none; 1: mild, 2: moderate, 3: obvious, and 4: very significant. The degree of erythema is scored using a scoring scaling based on erythema intensity. A cumulative score (i.e., erythema + scaling + thickening) can be used as an indicator of the severity of inflammation (the cumulative score is in a range of 0-12).

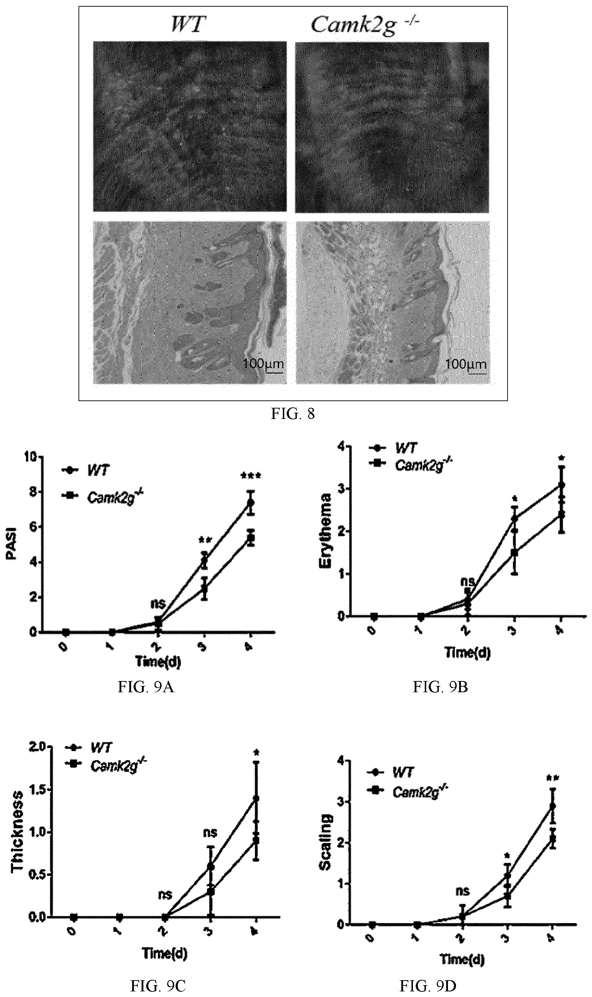

The results of skin section H&E staining and PASI score show that the psoriasis-like phenotype of IMQ-induced *Camk2g*^{*-*/*-*} mice is significantly weakened compared with that of IMQ-induced WT mice, reflecting the reduction of dorsal skin thickness and the reduction of erythema and scaling (FIGS. 8 and 9A-9D).

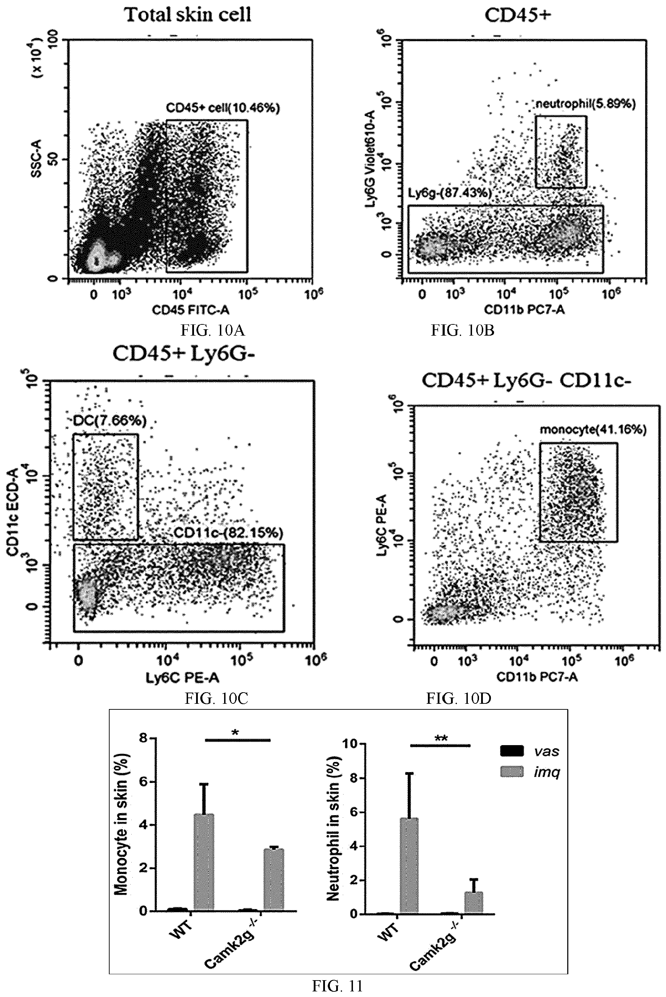

The isolated mouse skin immunocytes are clustered with specific labeled antibodies, in which monocytes and neutrophils are typical inflammatory infiltrating cells in psoriatic lesions (FIGS. 10A-10D). Flow cytometry shows that inflammatory infiltration in the dorsal skin of IMQ-induced *Camk2g*^{*-*/*-*} mice is significantly reduced (FIG. 11).

Separation steps of skin immunocytes include steps as follows. the skin is fully chopped and then digested in digestive enzymes at 37 °C and 225 revolutions per minute (rpm) for 1.5 hours to thereby obtain an initial digestion solution. The digestive enzymes are formulated as follows: 1.2 grams (g) bovine serum albumin (BSA) (Biofoxx, 4240GR100), 0.12 g type I collagenase (Sigma, C0130-5G), 0.06 g type IV collagenase (Worthington, LS004189) and 0.06 g hyaluronidase (Sigma, H3506) -5G) are dissolved in 30 milliliters (mL) dulbecco's modified eagle medium (DMEM) (HyClone, SH30022.01). 150 microliters (µL) of 1 microgram per milliliter (mg/mL) deoxyribonuclease (DNase) (Yuanye, S10073) is added in the initial digestion solution and the digestion is continued for 30 minutes to obtain a first cell suspension. After the digestion, the first cell suspension is filtered through a 70-µm filter. The filtered first cell suspension is centrifuged at 4 °C and 2000 rpm for 8 minutes, and first cell particles are collected after removing a first supernatant. The first cell particles are suspended in 6 mL of 40% lymphocyte isolation solution (5.4mL Percoll (GE Healthcare, 17-0891-09) +520µL Hank's Solution (Beyotime, C0219) +44µL 2×Hepes Buffer (Solarbio, H1080-100) +8.946 mL phosphate-buffered saline (PBS)) of a centrifuge tube. Then, 6 mL of 80% lymphocyte isolation solution (5.4 mL Percoll+520 µL Hank's Solution+44 µL 2×Hepes Buffer+1.491 mL PBS) is slowly added to the tube wall to obtain a first mixed solution, and the first mixed solution is clearly separated into two layers. The first mixed solution is centrifuged at 4 °C and 2000 rpm for 20 minutes, and the centrifugal lifting force is kept to a minimum. The liquid in the centrifuge tube after centrifugation can be divided into four layers. From top to bottom of the centrifuge tube, there are 40% lymphocyte isolation solution, lymphocytes, 80% lymphocyte isolation solution and cell fragments. Lymphocytes are carefully transferred into a new centrifuge tube and thoroughly washed with 10 mL PBS to obtain a second mixed solution. The second mixed solution is centrifuged at 4 °C and 2000 rpm for 8 minutes to obtain a second cell suspension, and second cell particles are collected after removing a second supernatant. Then, 1 µL of each cell membrane surface antibody is added to 50 µL of PBS, mixed with the second cell particles, and incubated at room temperature for 30 minutes in the dark. The incubated cells are washed with 3 mL PBS to obtain a third mixed solution, the third mixed solution is centrifuged at 4 °C and 2000 rpm for 5minutes to obtain a third cell suspension, third cell particles are collected after removing a third supernatant of the third cell suspension, and the third cell particles are suspended in 300-600 µL PBS according to a cell volume before detection. If staining with intracellular antibodies is required, 1 mL of cell permeabilizer (Fixation /Permeabilization concentrate (Invitrogen, 00-5123-43): eBioscience Fixation/Perm Diluent (Invitrogen, 00-5223-56) =1:3) is directly added to the cell pellet, and incubated at room temperature for 60 minutes in the dark. An appropriate amount of intracellular antibody is added to 50 µL of PBS, mixed with the cells, and protected from light at room temperature for 40 minutes. The cells are washed with 3 mL PBS and centrifuged at 4 °C and 2000 rpm for 5 minutes, and the cell particles are collected after removing the supernatant. The cells are suspended in 300-600 µL PBS according to the cell volume before detection.

The cell membrane surface antibodies include: CD45-FITC (eBioscience, REF11-0451-82), CD11b PE-Cy7 (BD, 552850), LY-6G-BV605 (BD, 563005), Ly-6C PE (BD, 560592), and CD11C PE-CF594 (BD, 562454).

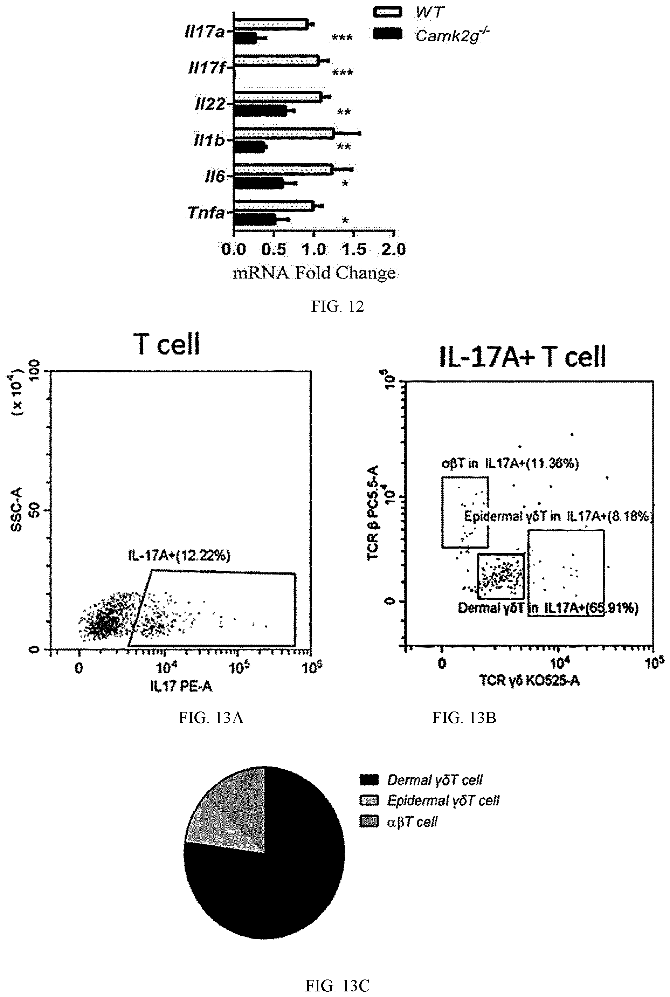

Quantitative PCR is performed on some proinflammatory genes known to be closely related to psoriasis and highly expressed in psoriasis lesions, including Il17a, Il17f, Il6, I122, IL1b and Tnfa. The results show that the expression of these marker genes is significantly decreased in the skin of *Camk2g*^{*-*/*-*} mice (FIG. 12).

### Example 5 Regulation of CAMK2γ on γδ T-17 cells in skin tissue of psoriasis mice

The recruitment of neutrophils and monocytes to psoriatic lesions is characteristic of psoriasis. The existing literature discloses that neutrophils and monocytes are driven by the key effector cytokine IL-17. IL-17 can be released by various cell types, such as Th17 cells, Tc17 cells, γδ T-17 cells, ILC3, etc.

The detection by flow cytometry is performed on the dorsal skin of IMQ-treated WT mice. The detection results show that in the dorsal skin of IMQ-treated WT mice, IL-17A is mainly produced by dermal γδ T cells and is rarely expressed in epidermal γδ T cells and αβ T cells (FIGS. 13A-13C).

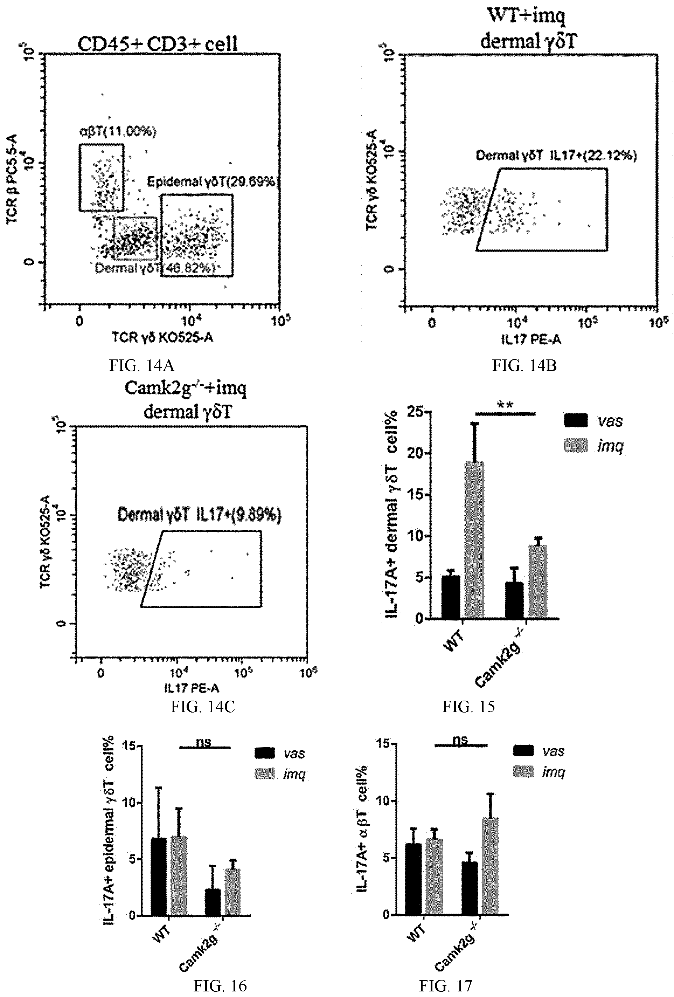

In order to study whether CAMK2y affects IL-17 production in IMQ-treated mouse skin, a proportion of IL-17+ cells in different skin T cell subsets are analyzed by flow cytometry, and anlysiss results are shown in FIGS. 14A-14C. In IMQ-treated *Camk2g*^{*-*/*-*} mice, IL-17A+ dermal γδ T cells are significantly lower than those in IMQ-treated WT mice (FIG. 15), while IL-17A+ epidermal γδ T cells and IL-17A+ αβ T cells remain unchanged (FIGS. 16-17).

### Example 6 Regulation of CAMK2γ+ sympathetic nerves on γδT-17 cells in skin tissue

The existing literature discloses that tyrosine hydroxylase (TH) is the rate-limiting enzyme in the biosynthesis of catecholamines (including dopamine (DA), norepinephrine (NE) and epinephrine), which is used to identify the sympathetic nerve in the skin. CaMKII family members participate in the regulation of catecholamine biosynthesis by stimulating TH phosphorylation in some neuronal populations.

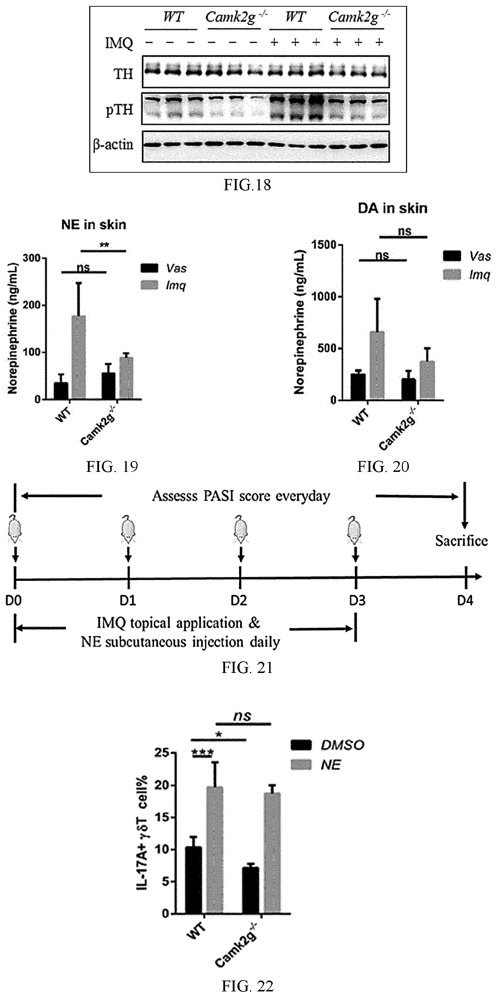

IMQ treatment results in a significant upregulation of TH phosphorylation in mouse skin while CAMK2G deletion attenuates this enhanced effect as detected by western blot (FIG. 18).

The secretion of catecholamine neurotransmitter in mouse skin is detected by enzyme linked immunosorbent assay (ELISA), and it is found that the NE level in the skin of IMQ-treated *Camk2g*^{*-*/*-*} mice is lower than that of WT mice, but there is no difference in dopamine in the skin (FIGS. 19-20).

Subcutaneous injection of NE aggravates the IMQ-induced psoriasis-like phenotype and increases a proportion of IL-17A+ γδ T cells in mouse skin, and more importantly, administration of NE counteracts the decrease of IL-17A+ γδ T cells caused by CAMK2G deletion (FIGS. 21-23), demonstrating that sympathetic nerves are indeed the main source of NE in the skin.

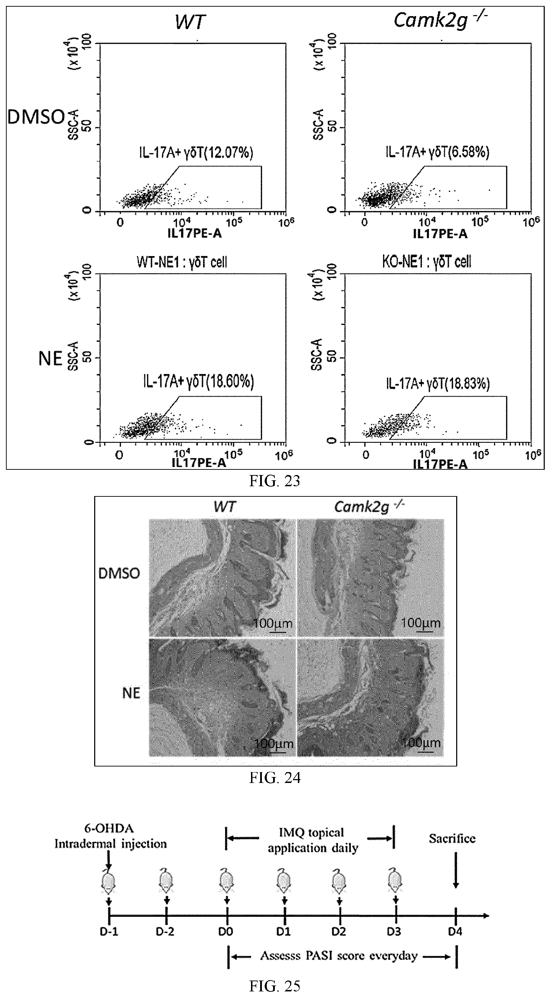

The difference in skin thickness between WT mice and IMQ-induced *Camk2g*^{*-*/*-*} mice disappeared after injection of NE (FIG. 24). These evidences suggest that the regulation of IMQ-induced skin inflammation by CAMK2y may be related to the secretion of NE by skin sympathetic nerves, and CAMK2 y+ sympathetic nerves in skin tissue locally regulate IL-17A production in γδ T cells by secreting NE.

### Example 7 Effect of removing skin sympathetic nerves on psoriasis phenotype of a mouse model

The existing literature discloses that 6-hydroxydopamine (6-OHDA) is a selective neurotoxin of catecholaminergic neurons, which is used to remove sympathetic nerves. Intraperitoneal injection of 6-OHDA can reduce IMQ-induced ear swelling, but does not affect the production of IL-17, which is attributed to cardiovascular effects and/or systemic immune disorders caused by systemic sympathectomy.

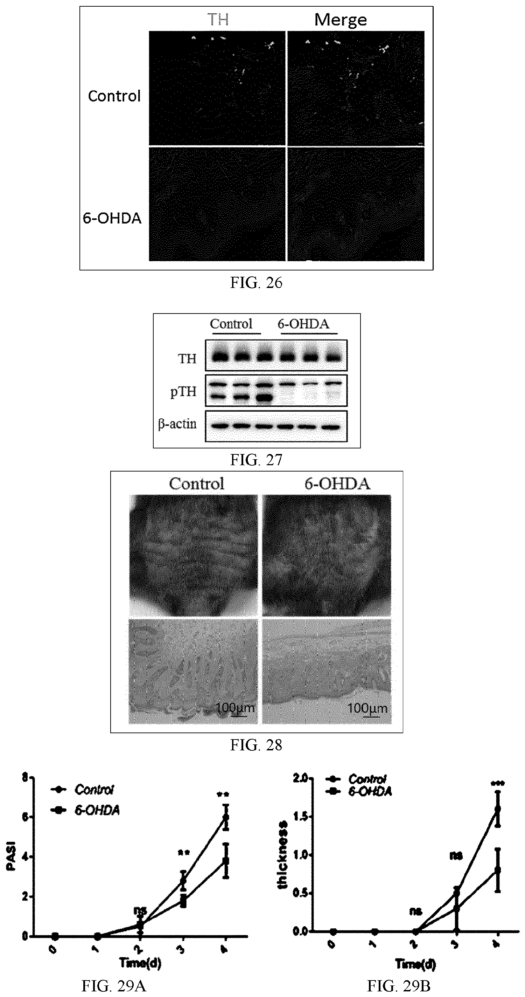

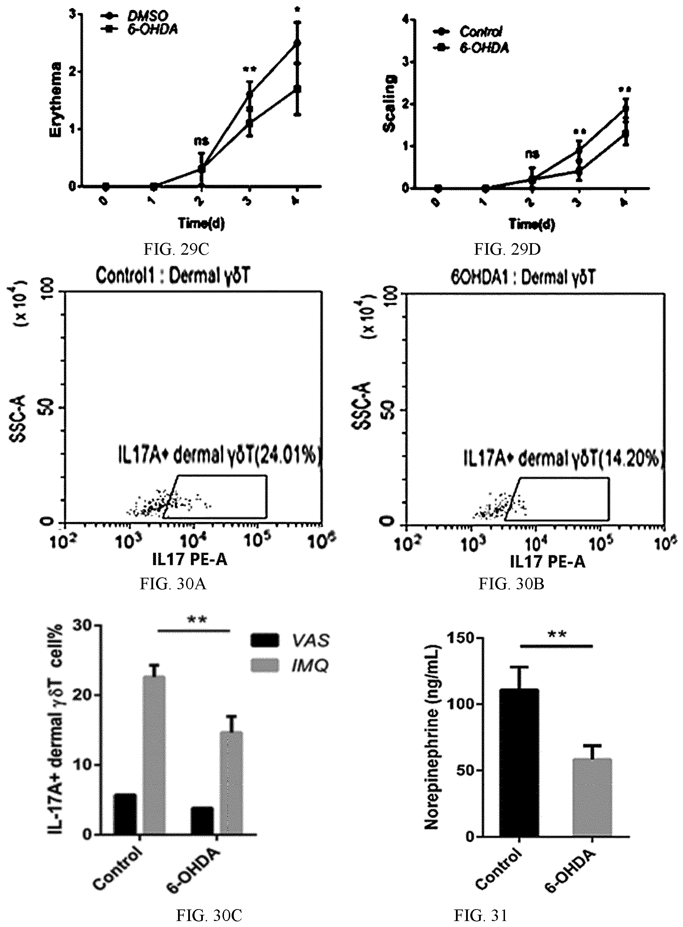

In order to observe the effect of local sympathectomy on psoriasis mice, mice are injected with 6-OHDA intradermally before IMQ treatment (FIG. 25). The significant reduction of TH and pTH (parathyroid hormone) by immunofluorescence and western blot confirms the inactivation of local sympathetic nerves in the skin (FIGS. 26-27). Subsequent studies demonstrates that skin sympathetic denervation reduces IMQ-induced psoriasis-like phenotype in mice (FIGS. 28, 29A-29D). In addition, IL-17A+ γδ T cells are reduced in sympathetic denervated skin, suggesting that skin sympathetic nerves influence psoriasis-like skin inflammation by regulating IL-17 production by γδ T cells (FIGS. 30A-30C). The reduction of NE in sympathetic denervated skin suggests that sympathetic nerves may regulate IL-17 production by γδ T cells by secreting NE (FIG. 31).

### Example 8 Effect of CAMK2G gene knockout on SH-SY5Y cells damaged by 6-OHDA

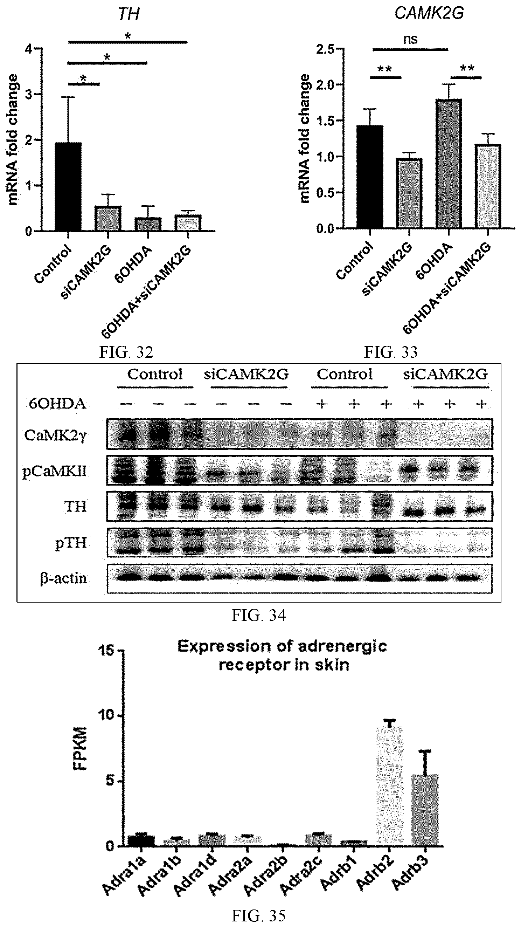

To further study whether CAMK2y directly affects the expression of TH in sympathetic nerves, RNA interference (RNAi) method is used to knock down CAMK2G in human neuroblastoma cell (SH-SY5Y). SH-SY5Y is a poorly differentiated tumor cell with high similarity in morphology, physiology and biochemical function to dopaminergic neurons in real-time *in vivo,* and can constitutively express TH. The results detected by qPCR show that 6-OHDA induced SH-SY5Y cell injury could lead to the decrease of intracellular TH transcription level, and knockdown of CAMK2G gene could also reduce the TH transcription level (FIG. 32). 6-OHDA induced SH-SY5Y cell injury had no significant effect on CAMK2G expression (FIG. 33). The results analyzed by western blot show that knockout of CAMK2G gene could significantly reduce the total protein level and pTH level of CAMK2y, 6-OHDA induced SH-SY5Y cell injury also reduces the total protein level and pTH level of CAMK2y, and 6-OHDA induced SH-SY5Y cell injury may lead to increased phosphorylation levels of other members of the CaMKII family (FIG. 34).

### Example 9 Expression patterns of NE receptors in skin

RNA sequencing (RNA-seq) of skin tissue from IMQ-induced psoriasis mice reveals that beta 2 adrenergic receptor (ADRB2) (β2-AR) is the predominant NE receptor expressed in skin (FIG. 35).

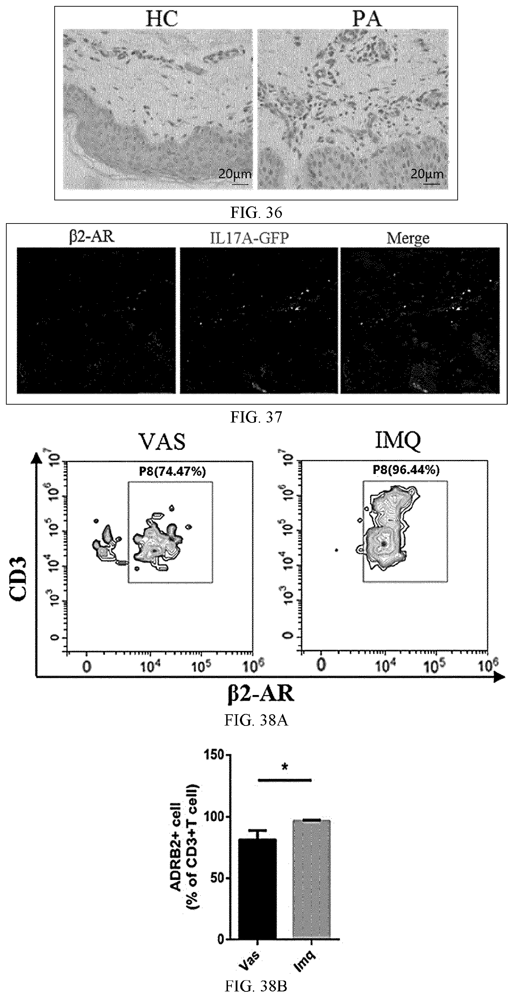

The skin lesions of psoriasis patients and healthy control skin are analyzed by immunohistochemistry, and the results show that, β2-AR is expressed on immunocytes infiltrating the dermis (FIG. 36).

Through immunofluorescence and flow cytometry analysis, it is found that most of the cells expressing IL-17 express β2-AR (FIG. 37), IMQ treatment increases a proportion of β2-AR+ T cells in mouse skin (FIGS. 38A-38B).

### Example 10 Regulation of β2-AR activators on psoriasis phenotype in a mouse model

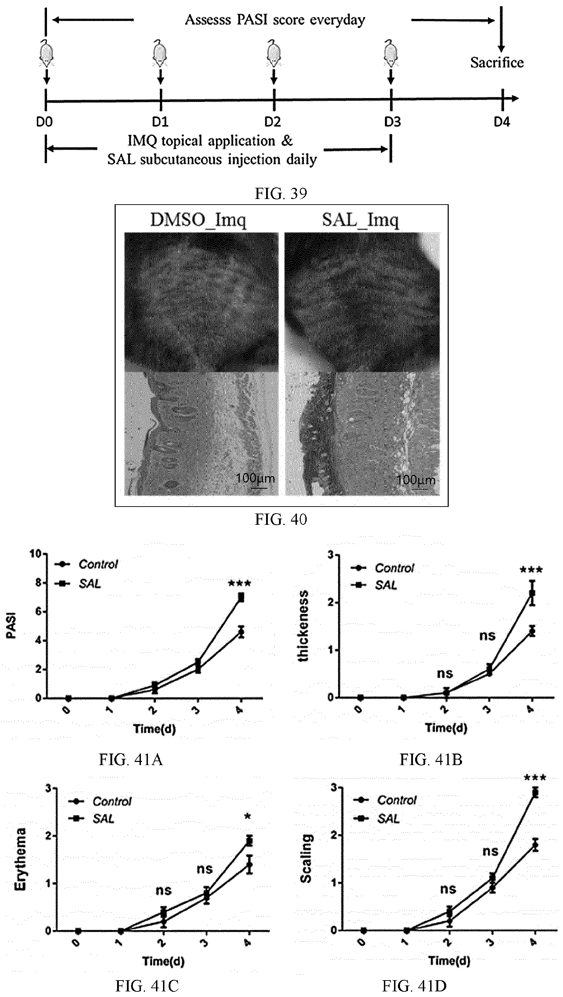

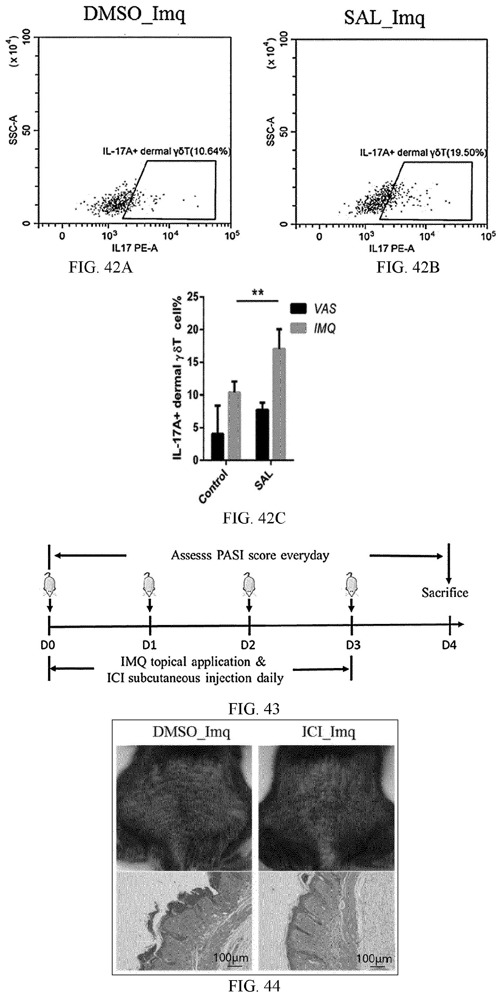

In order to further study whether NE directly affects γδ T-17 cells through β2-AR, salmeterol xinafoate (SAL), a selective ADRB2 agonist) is subcutaneously injected into IMQ-induced mice (FIG. 39). The results show that compared with the control group, the psoriasis phenotype of mice injected with SAL increased, including increased dorsal skin thickness and increased erythema and scaling (FIGS. 40, 41A-41D). In IMQ-induced mouse skin, injection of SAL promotes IL-17A production by γδ T cells (FIGS. 42A-42C).

### Example 11 Regulation of β2-AR antagonists on psoriasis phenotype in a mouse model

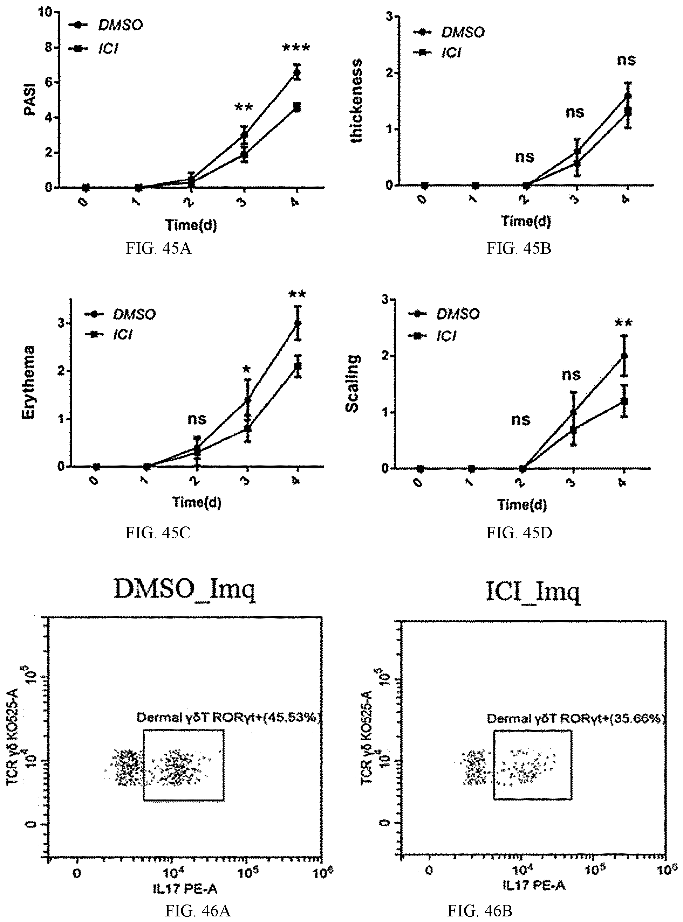

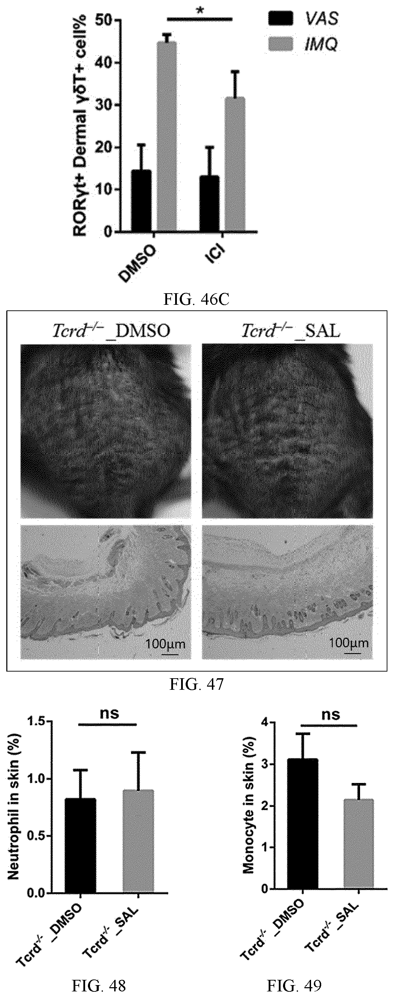

In an embodiment of the invention, ICI 118551 (ICI, a selective ADRB2 antagonist) is injected subcutaneously into IMQ-induced mice (FIG. 43). The results show that the psoriasis phenotype of mice injected with ICI is reduced compared with the control group (FIGS. 44, 45A-45D). In IMQ-induced mouse skin, injection of ICI reduces a proportion of skin γδ T-17 cells (FIGS. 46A-46C).

### Example 12 ne-adrb2 signal pathway regulation yδ T cells secrete IL-17

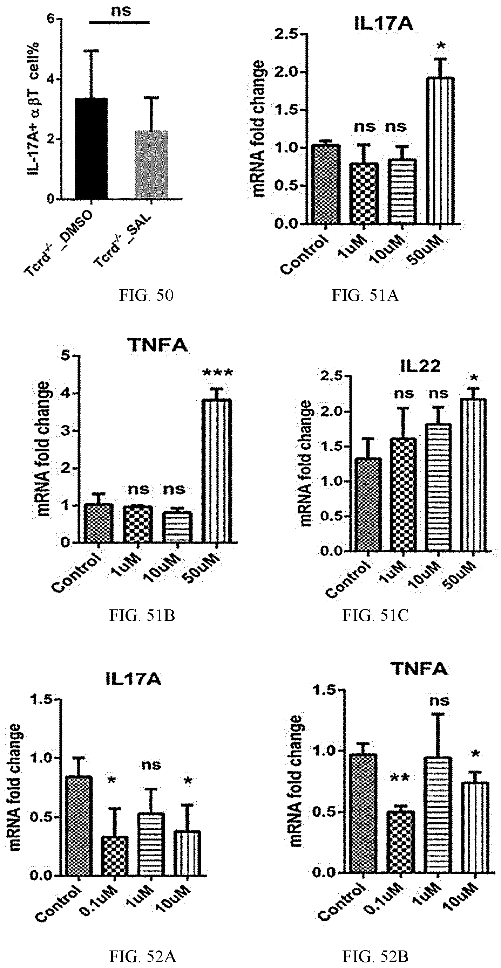

γδ T cell deficient-mice (*Terd*^{*-*/*-*}) are injected subcutaneously with SAL, and it is found that the promotion of psoriasis phenotype and inflammatory infiltration by SAL is counteracted by the lack of γδ T cells (FIGS. 47-49). SAL has no effect on the ability of αβ T cells to produce IL-17 (FIG. 50). Combined with the previous results, NE in the skin mainly derives from the skin sympathetic nerves and mediates the release of IL-17 from γδ T cells instead of αβ T cells through the NE-ADRB2 signaling pathway.

### Example 13 Validation of the immunomodulatory effects of β2-AR in a cell model

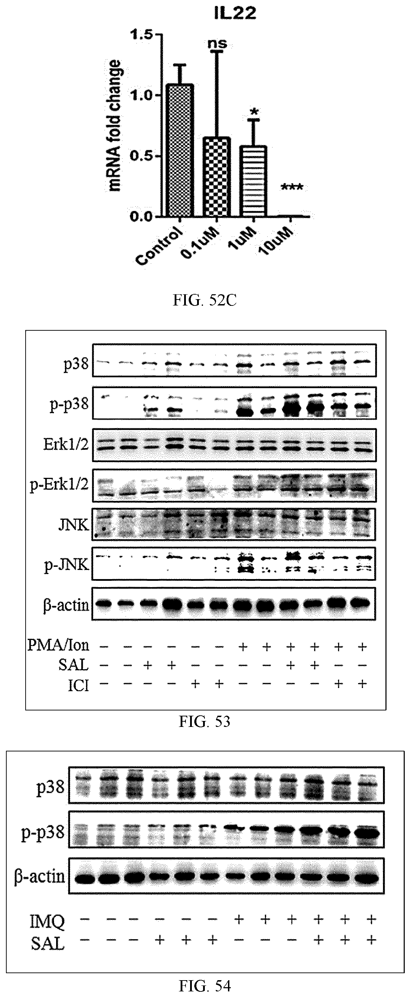

In order to in order to study how ADRB2 regulates the inflammatory response of γδ T cells, Jurkat cells (a kind of immortal human T lymphocytes) are stimulated with ADRB2 agonist SAL and antagonist ICI 118551 respectively. High concentrations of SAL increase the production of T-cell proinflammatory cytokines IL-17A, TNF-α and IL-22 upon co-stimulation with PMA and ionomycin (Ion) (FIGS. 51A-51C). In contrast, ICI reduces the production of the T-cell proinflammatory cytokines (FIGS. 52A-52C).

### Example 14 Validation of NE-ADRB2-p38 signaling pathway

In Jurkat T cells activated by PMA/ionomycin for 2 hours, p38 (also referred to as p38 mitogen-activated protein kinase (p38 MAPK)) phosphorylation is significantly increased by SAL stimulation for 4 hours, while p38 phosphorylation is inhibited by ICI treatment for 4 hours, which does not affect the activation of extracellular signal-regulated kinase (ERK) and c-Jun N-Terminal Kinase (JNK), two other members of mitogen-activated protein kinase (MAPK) family (FIG. 53). It suggests that ADRB2 regulates T cell activation, which depends on p38 phosphorylation rather than ERK and JNK. This is consistent with the existing literature that p38 phosphorylation promotes the proliferation of Th17 cells in autoimmune arthritis. P38 MAPK signaling pathway plays a role in the activation and differentiation of T cells in the peripheral blood immune system.

The p38 level in the mouse skin of PMA/ion activated Jurkat T cells is detected, and it is found that p38 phosphorylation is activated by IMQ. SAL treatment enhanced the activation of p38 in IMQ-induced skin, while ICI treatment showed inhibitory effect (FIGS. 54-55).

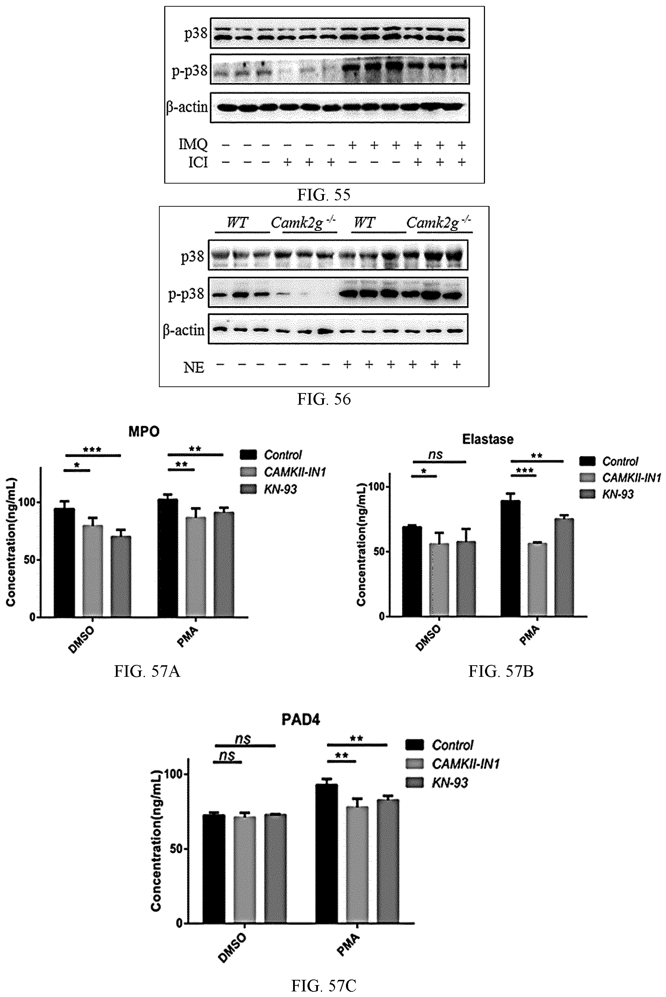

p38 phosphorylation is inhibited in the skin of *Camk2g*^{*-*/*-*} mice following PMA/ion activation of Jurkat T cells compared to IMQ-treated WT mice. NE injection increases and restores p38 phosphorylation (FIG. 56).

These results show that CAMK2y promotes IL-17 production by T cells by activating the NE-ADRB2-p38 signaling pathway.

### Example 15 By promoting synthesis of nicotinamide adenine dinucleotide phosphate (NAPDH) oxidase complex subunit protein, CAMK2γ stimulates production of ROS in neutrophils, so as to regulate formation of NETs

The existing literature discloses that although T cells producing IL-17 have long been the mainstream of psoriasis immunology research, neutrophils, as the largest number of cells in the innate immune system, have attracted much attention in the occurrence and development of psoriasis. Activated neutrophils produce enhanced respiratory burst, accompanied by the production of reactive oxygen species (ROS), ultimately leading to the development of NETosis. NETosis is a form of neutrophil death different from apoptosis and necrosis. NETosis is accompanied by the formation of NETs. NETs release proteases such as myeloperoxidase (MPO), neutrophil elastase (NE) and peptidyl arginine deiminase 4 (PAD4), which are involved in the hydrolysis and activation of inflammatory mediators and the formation of NETs of psoriasis autoantigens. HL60 is a bone marrow cell line with the potential to differentiate into neutrophils stimulated by dimethyl sulfoxide (DMSO). CAMK2y is the member of CAMK family with the highest expression level and activity in HL-60 cells.

In order to study whether CAMK2y is involved in the formation of NETs in activated neutrophils, two kinds of CaMKII inhibitors, CaMKII-IN1 and KN93, are used. The results show that both CaMKII-IN1 and KN93 inhibits the release of MPO, elastase and PAD4 in PMA-activated HL-60 cells, indicating that inhibition of CAMK2y activity in neutrophils can attenuate the formation of NETs (FIGS. 57A-57C).

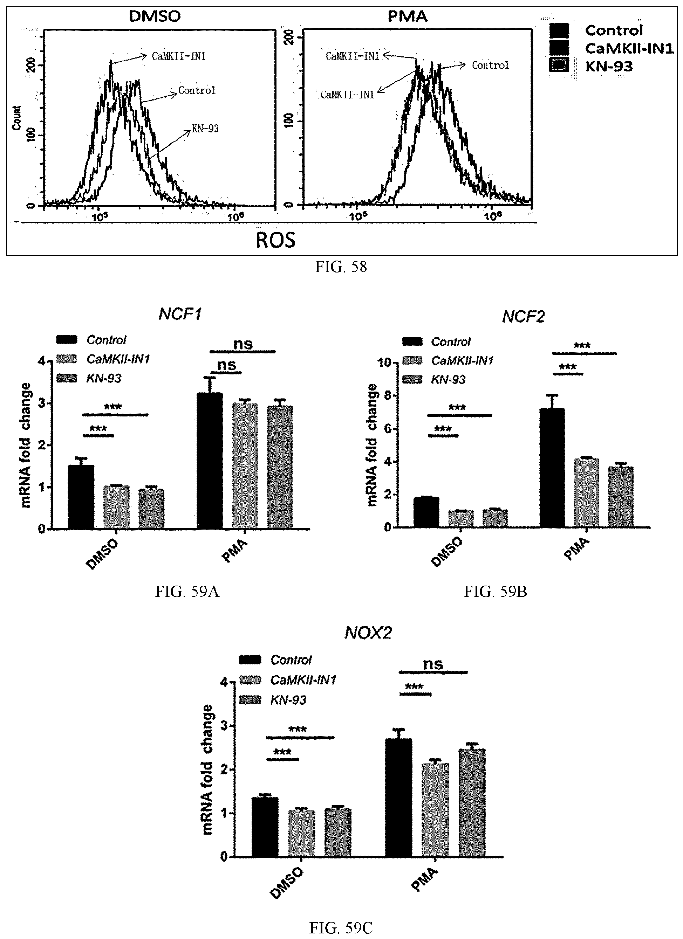

It is also found that inhibition of CAMK2y activity reduces the production of intracellular ROS (FIG. 58) and the mRNA levels of NCF1, NCF2 and NOX2 (FIGS. 59A-59C). These mRNAs can produce the NAPDH oxidase complex protein subunit, which has been found to be a major source of intracellular ROS.

These results show that the activated CAMK2y stimulates the production of ROS in neutrophils by promoting the synthesis of NAPDH oxidase complex subunit protein, so as to regulate the formation of NETs. the mechanism by which CAMK2y in the circulatory system is involved in the progression of psoriasis is explained.

## Claims

1. A medicament for use in a method of treating psoriasis, comprising a beta 2 adrenergic receptor (ADRB2) inhibitor, wherein the ADRB2 inhibitor is ICI 118551.

## Patentansprüche

1. Ein Arzneimittel zur Verwendung in einer Methode zur Behandlung von Schuppenflechte, das einen Beta-2-Adrenerger-Rezeptor (ADRB2)-Inhibitor enthält, wobei der ADRB2-Inhibitor ICI 118551 ist.

## Revendications

1. Un médicament à utiliser dans une méthode de traitement de la psoriasis, comprenant un inhibiteur du récepteur bêta-2 adrénergique (ADRB2), **caractérisé en ce que** l'inhibiteur ADRB2 est l'ICI 118551.
